(19) Europäisches Patentamt
European Patent Office
Office européen des brevets

(11) EP 3 640 270 A1

(12) **EUROPEAN PATENT APPLICATION**
published in accordance with Art. 153(4) EPC

(43) Date of publication:
**22.04.2020 Bulletin 2020/17**

(21) Application number: **17908691.3**

(22) Date of filing: **05.05.2017**

(51) Int Cl.:
***C08F 220/06*** *(2006.01)* ***C08F 220/10*** *(2006.01)*
***C08B 37/16*** *(2006.01)* ***C08J 3/075*** *(2006.01)*
***G02C 7/04*** *(2006.01)* ***G02B 1/04*** *(2006.01)*
***A61K 31/385*** *(2006.01)*

(86) International application number:
**PCT/ES2017/070282**

(87) International publication number:
**WO 2018/202925 (08.11.2018 Gazette 2018/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Universidade de Santiago de Compostela**
**15782 Santiago de Compostela (A Coruña) (ES)**

(72) Inventors:
- **ÁLVAREZ LORENZO, Carmen Isabel**
**E-15782 Santiago de Compostela (A Coruña) (ES)**
- **CONCHEIRO NINE, Ángel**
**E-15782 Santiago de Compostela (A Coruña) (ES)**

(74) Representative: **ABG Intellectual Property Law, S.L.**
**Avenida de Burgos, 16D**
**Edificio Euromor**
**28036 Madrid (ES)**

(54) **LIPOIC ACID HYDROGELS**

(57) The present invention relates to acrylic hydrogels functionalized with cyclodextrin that comprises α-lipoic acid, to methods for obtaining said hydrogels, and to the use of contact lenses prepared from said hydrogels as demulcent agents.

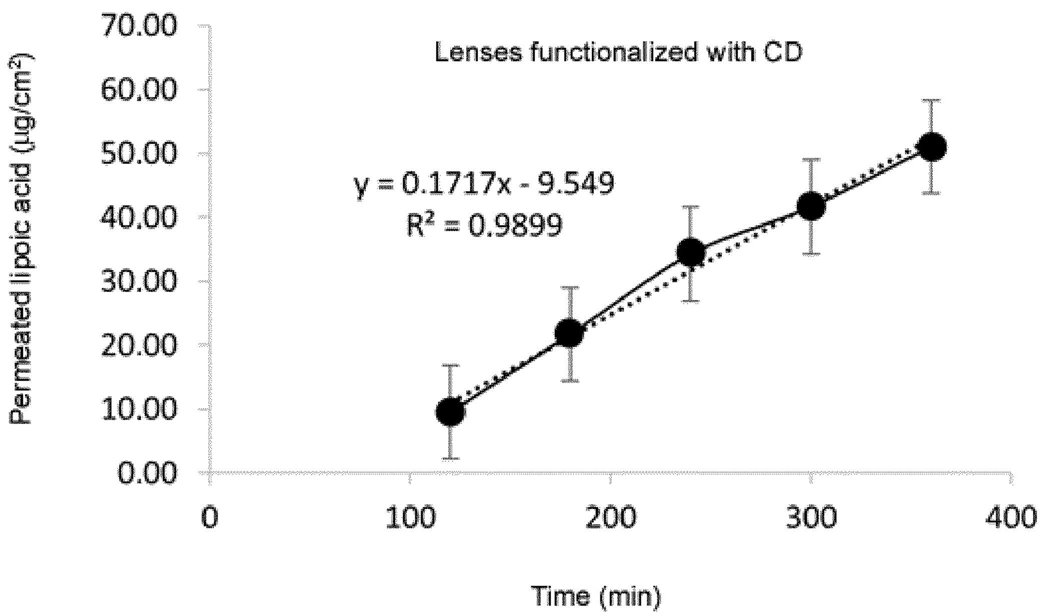


Fig. 5

EP 3 640 270 A1

## Description

### Field of the Invention

The present invention relates to acrylic hydrogels functionalized with cyclodextrin, to methods for obtaining said hydrogels, as well as the medical, particularly ophthalmic, use of said hydrogels.

### Background of the Invention

Dryness of the eye is a condition in which a person's eye is not exposed to tears in a sufficient amount or with a sufficient quality to lubricate and nourish the eye. Tears are needed to maintain the health of the front surface of the eye and to provide clear vision. With each blink, tears are spread over the front surface of the eye, known as the cornea. Tears provide lubrication, reduce the risk of eye infection, clean foreign matter out of the eye, and keep the surface of the eyes smooth and clear.

Dryness of the eyes has a considerably high prevalence, affecting about 17% of the population in China, whereas in the United States, that number reaches up to 22%. The figures of affected individuals in diabetic populations are particularly dramatic, and can reach even up to 50% of the population.

In some cases, dry eyes can progress to irritation or even considerable inflammation, and in the worst cases to scarring of the front surface of the eye, which seriously affects the quality of life of patients.

A class of compounds used for the treatment of dryness of the eyes comprises demulcents. The United States Food and Drug Administration (FDA) classifies demulcents as agents which protect and lubricate mucous membrane surfaces and relieve dryness and irritation.

A series of demulcents are known, for example cellulose derivatives such as carboxymethylcellulose (CMC), hydroxyethylcellulose (HEC), hydroxypropylcellulose (HPC), or methylcellulose (MC), dextran, gelatin, α-lipoic acid (ALA), polyols such as glycerin, polyethylene glycol (PEG), polysorbate 80, propylene glycol, polyvinyl alcohol (PVA), and polyvinylpyrrolidone (PVP, povidone).

In particular, ALA is an organosulfur compound derived from octanoic acid.

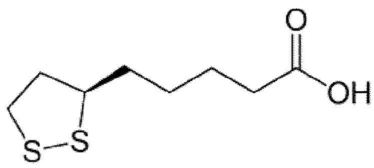

α-lipoic acid (ALA)

α-Lipoic acid is marketed as an oral nutritional supplement for the treatment and prophylaxis of diabetic neuropathy (Thioctacid 600 HR), and also in the form of ophthalmic solutions comprising 0.1% of the organosulfur compound (Tioretin A, CE Marking 0373; BIOOS Italy) under the indication of sustaining tear fluid production. It is this latter capacity of ALA that gives it demulcent properties. In connection with this, Andrade et al. (Exp Eye Res. 2014, 120:1-9) reported that ALA disrupts the metabolism of reactive nitrogen species, which have a direct impact on tear production. More specifically, it was observed that ALA increases peroxidase activity and produces an increase in tear production.

Demulcents are administered in the eye usually in the form of solutions or gels. Contact lenses comprising demulcent agents have more recently been developed. Patent document EP 2 314 640 A1 discloses hydrogels and contact lenses produced from said hydrogels which can be loaded with demulcents.

A determining factor with regard to the demulcent efficacy of contact lenses is the level of loading of the demulcent agent in the contact lens. Therefore, the object of present invention is the development of enhanced demulcent agents, particularly enhanced contact lenses, capable of holding large amounts of demulcent and in turn being capable of suitably releasing the demulcent agent in the eye.

### Summary of the Invention

The authors of the present invention have unexpectedly discovered that the formulation of ALA with specific hydrogels allows preparing contact lenses with a surprisingly high load of the demulcent agent, and that said load is much higher than the load observed with other demulcent agents.

Therefore, in a first aspect the present invention relates to a hydrogel in the form of a three-dimensional network, characterized in that it comprises crosslinked acrylic or methacrylic chains, wherein the chains comprise alkyl groups

to which cyclodextrins are attached by means of an ether bond; and wherein the hydrogel further comprises α-lipoic acid, or a pharmaceutically acceptable salt thereof.

A second aspect of the invention relates to a method for preparing three-dimensional acrylic hydrogels with pendant cyclodextrins of the first aspect of the invention by means of a method comprising the steps of:

a. polymerizing and crosslinking a mixture comprising i) monofunctionalized acrylic or methacrylic monomers, and ii) acrylic or methacrylic monomers comprising glycidyl groups, to form a base framework of a three-dimensional network; and
b. reacting cyclodextrin with the base framework;

wherein α-lipoic acid is added either to the hydrogel after step b., or to the cyclodextrin before carrying out step b.

In another aspect, the present invention relates to a hydrogel obtainable by means of a method as described in the second aspect of the invention.

In another aspect, the present invention relates to a contact lens comprising a hydrogel as described in the first aspect of the invention. In another aspect, the present invention relates to a method for preparing said contact lens.

In an additional aspect, the present invention relates to the use of a hydrogel, and more specifically a contact lens, as described in the previous aspects, as a demulcent. More specifically, said hydrogel or contact lens is used to treat or prevent dryness of the eye, and even more particularly to treat or prevent the discomfort associated with the use of contact lenses.

## Description of the Drawings

Figure 1. Transfer profiles of ALA in STF, at 35°C, from hydrogels loaded by dipping in an aqueous solution of the demulcent. SCL-αCD: hydrogel functionalized with αCD; SCL-βCD: hydrogel functionalized with βCD; SCL: unfunctionalized hydrogel.
Figure 2. Transcorneal penetration of ALA according to the time when ALA was applied on bovine corneas in the form of a solution (ALA), or incorporated in hydrogels without CD (SCL) or with pendant βCD (SCL-βCD).
Figure 3. Amount of ALA initially applied on the corneas, amounts accumulated in the receiving medium according to the time, amount remaining in the donor chamber at 6 h, amount of ALA in the corneas after 6 h, and amount remaining in the hydrogels after 6 h.
Figure 4. Transcorneal penetration of ALA according to the time when ALA was applied on bovine corneas in the form of a solution, or incorporated in contact lenses with pendant βCD (SCL-βCD).
Figure 5. Determination of ALA flow through the cornea when ALA was applied on bovine corneas incorporated in contact lenses with pendant βCD (SCL-βCD).
Figure 6. Determination of ALA flow through the cornea when ALA was applied on bovine corneas in the form of a solution.

## Detailed Description of the Invention

In one aspect, the present invention relates to a hydrogel in the form of a three-dimensional network characterized in that it comprises or is formed by crosslinked acrylic or methacrylic chains, wherein the chains comprise or have alkyl groups to which cyclodextrins are attached by means of an ether bond; and wherein the hydrogel further comprises α-lipoic acid, or a pharmaceutically acceptable salt thereof. These hydrogels shall be referred to as three-dimensional acrylic hydrogels with pendant cyclodextrins to better understand the specification.

In the present invention, acrylic or methacrylic chain is understood to mean a polymer chain which is the result of the polymerization of acrylic or methacrylic monomers.

Acrylic or methacrylic unit is understood to mean each monomer unit forming the polymer chain after the polymerization of acrylic or methacrylic monomers.

Monofunctionalized acrylic or methacrylic units are the result of the polymerization of monomers containing a single acrylic or methacrylic group. Bifunctionalized acrylic or methacrylic units are the result of the polymerization of monomers containing two or more acrylic or methacrylic groups.

In a preferred embodiment, the acrylic or methacrylic chains of the hydrogels of the invention comprise or are formed by acrylic or methacrylic units having an alkyl ether group; bifunctionalized acrylic or methacrylic units; and monofunctionalized acrylic or methacrylic units. In one embodiment, the chains do not comprise another type of unit.

In a preferred embodiment, these three-dimensional acrylic hydrogels with pendant cyclodextrins are characterized in that the proportion by weight of the acrylic or methacrylic units containing an alkyl ether group is preferably

between 0.1% and 10% with respect to the weight of the hydrogel. In another more particular aspect, the proportion by weight of the bifunctionalized acrylic or methacrylic units is preferably between 0.1% and 10% with respect to the weight of the hydrogel.

Strictly speaking, the three-dimensional character is provided by the three-dimensional network (also referred to herein as framework) formed by the acrylic or methacrylic chains after carrying out the polymerization of the different monomers (see step a. of the method of the invention). However, given that said network is comprised in the hydrogel, the hydrogel itself can also be considered a three-dimensional acrylic hydrogel.

The three-dimensional acrylic hydrogels with pendant cyclodextrins of the present invention have the capacity to incorporate water in large proportions without dissolving, giving rise to viscoelastic systems provided with high optical clarity. This optical clarity, combined with physical and mechanical properties of the hydrogels, as well as the excellent biocompatibility of the cyclodextrins and the acrylic frameworks of the hydrogel, render the hydrogels of the present invention useful as components in contact lenses, and particularly soft contact lenses.

In one embodiment, the cyclodextrin is preferably selected from $\alpha$-, $\beta$- or $\gamma$-cyclodextrin, a cyclodextrin formed by more than eight units of $\alpha$-1,4-glucopyranose, or a linear or branched alkyl derivative, linear or branched hydroxyalkyl derivative, acetyl-, propionyl-, butyryl-, succinyl-, benzoyl-, palmityl-, toluenesulfonyl-, acetyl alkyl, glucosyl-, maltosyl-, carboxymethyl ether-, carboxymethyl alkyl-, phosphate ester-, 3-trimethylammonium-, sulfobutyl ether-cyclodextrin, or a cyclodextrin polymer, or combinations thereof.

More preferably, the cyclodextrin is selected from $\alpha$- or $\beta$-cyclodextrin, or combinations thereof. In a particularly preferred embodiment, the cyclodextrin is $\alpha$-cyclodextrin. In another particularly preferred embodiment, the cyclodextrin is $\beta$-cyclodextrin.

In one embodiment, the proportion of cyclodextrin is comprised between 1 and 0.2 units of cyclodextrin for every alkyl ether group of the hydrogel.

In one embodiment, the ALA forms inclusion complexes with the cyclodextrin. In another embodiment, the ALA is furthermore dispersed in the hydrogel.

In one embodiment, the ALA is a pharmaceutically acceptable salt of ALA. Pharmaceutically acceptable salt is understood to mean any salt that is physiologically tolerated (usually meaning that it is not toxic, particularly as a result of the counterion) when used suitably for a treatment, applied or used particularly in human beings and/or mammals. Examples of pharmaceutically acceptable salts of ALA are the salts formed with ammonium hydroxide, basic amino acids optionally substituted with alkyl-or oxyalkylamines, alkylenediamines, saturated cyclic amino compounds, N-methylglucamine, creatine, and tromethamine.

In a particular embodiment, the ALA is the (R)-ALA stereoisomer. In another embodiment, the ALA is the (S)-ALA stereoisomer. In one embodiment, the ALA is a mixture of both stereoisomers. In a more particular embodiment, it is a racemic mixture.

The concentration of ALA in the hydrogel will vary depending on the use to be given to the hydrogel or contact lens. In a preferred embodiment, the concentration of ALA is at least the minimum concentration that allows a maximum load of the hydrogel, or at least the minimum concentration that allows maximum occupation of cyclodextrins.

In one embodiment, the concentration of ALA in the hydrogel is at least 25 mg of ALA for every 1 g of dry mass of hydrogel. In another embodiment, it is at least 35 mg of ALA for every 1 g of dry mass of hydrogel. In another embodiment, it is at least 45 mg of ALA for every 1 g of dry mass of hydrogel. In another embodiment, it is at least 55 mg of ALA for every 1 g of dry mass of hydrogel. In another embodiment, it is at least 65 mg of ALA for every 1 g of dry mass of hydrogel. In another embodiment, it is between 25 and 65 mg of ALA for every 1 g of dry mass of hydrogel. In another embodiment, it is between 35 and 65 mg of ALA for every 1 g of dry mass of hydrogel. In another embodiment, it is between 45 and 65 mg of ALA for every 1 g of dry mass of hydrogel. In another embodiment, it is between 55 and 65 mg of ALA for every 1 g of dry mass of hydrogel.

In the context of the present invention, weight or dry mass of hydrogel is understood to mean the weight or mass of the hydrogel after eliminating the aqueous phase by drying. Namely, the weight or mass of hydrogel resulting after the drying usually comprises 1% by weight of water or less.

In an additional aspect, the invention relates to a contact lens comprising the hydrogel of the invention as described in any of its embodiments.

In one embodiment, the contact lens is a soft contact lens. In the context of the present invention, a soft contact lens is a contact lens having an elastic modulus (i.e., Young's modulus) of less than 2.5 MPa.

In one embodiment, the hydrogel or the contact lens has a water content by weight with respect to the weight of the hydrogel or lens of 20% to 80%.

In one embodiment, the hydrogel or contact lens further comprises additional active agents which serve to treat dryness of the eye or a derived symptom such as inflammation.

In one embodiment, the additional active agent is a demulcent. More particularly, it is a demulcent which is selected from the group consisting of cellulose derivatives such as carboxymethylcellulose (CMC), hydroxyethylcellulose (HEC), hydroxypropylcellulose (HPC), hydroxypropylmethylcellulose (HMPC), or methylcellulose (MC), dextran, gelatin,

polyols such as glycerin, polyethylene glycol (PEG), polysorbate 80, propylene glycol, polyvinyl alcohol (PVA), and polyvinylpyrrolidone (PVP, povidone).

In one embodiment, the additional active agent is an anti-inflammatory agent. More particularly, the anti-inflammatory agent is selected from the group consisting of ibuprofen, ketoprofen, flurbiprofen, fenoprofen, naproxen, piroxicam, tenoxicam, isoxicam, meloxicam, indomethacin, aceclofenac, diclofenac, and a combination thereof.

Methods of Preparation

Another aspect of the invention relates to the preparation of the hydrogels of the invention by means of a method comprising the steps of:

a. polymerizing and crosslinking a mixture comprising i) monofunctionalized acrylic or methacrylic monomers, and ii) acrylic or methacrylic monomers comprising a glycidyl group, to form a base framework of a three-dimensional network; and
b. reacting cyclodextrin with the base framework;
wherein the ALA can be added either to the hydrogel after step b., or to the cyclodextrin before carrying out step b.

This method takes place under conditions that do not compromise the stability of ALA, and over the course of the method waste implying environmental pollution risks is not generated.

Step a. of the present invention is characterized in that polymerization is carried out in the absence of monomers comprising cyclodextrin in their structure.

Acrylic or methacrylic chains are formed through the polymerization of step a. The crosslinking of these chains as they grow can be achieved in a number of ways known to one skilled in the art. In a preferred embodiment, the crosslinking is achieved through the use of bifunctionalized acrylic or methacrylic monomers in step a. In the context of the present invention, reticulation and crosslinking mean the same thing.

Therefore, in a particular embodiment, the base framework of the network which is prepared in step a. is prepared by polymerization of acrylic or methacrylic monomers having or comprising a glycidyl group, monofunctionalized acrylic or methacrylic monomers, and bifunctionalized acrylic or methacrylic monomers. A base framework is thereby obtained, said base framework comprising acrylic or methacrylic chains comprising or being formed by acrylic or methacrylic units having or comprising a glycidyl group, bifunctionalized acrylic or methacrylic units, and monofunctionalized acrylic or methacrylic units.

The base framework which is prepared in step a. is a hydrogel itself.

The monomers giving rise to the acrylic or methacrylic units comprising a glycidyl group are preferably glycidyl acrylate or glycidyl methacrylate.

In one embodiment, the mixture of step a. has glycidyl acrylate or glycidyl methacrylate at a concentration of between 50 and 1000 mM, more preferably between 100 and 800 mM, even more preferably between 300 and 500 mM, and particularly 400 mM.

In a preferred embodiment, the base framework or hydrogel which is prepared in step a. has glycidyl acrylate or glycidyl methacrylate at a concentration of between 50 and 1000 mM, more preferably between 100 and 800 mM, even more preferably between 300 and 500 mM, and particularly 400 mM.

The monomers giving rise to acrylic or methacrylic units having an acrylic or methacrylic group in their structure (monofunctionalized) are preferably hydroxyethyl methacrylate, 1-(tristrimethylsiloxysilylpropyl)-methacrylate, methylmethacrylate, N,N-dimethylacrylamide, N,N-diethylacrylamide, methacrylic acid, acrylic acid, aminopropyl methacrylate, cyclohexyl methacrylate, or fluoro-siloxane acrylate.

The acrylic or methacrylic units having two or more acrylic or methacrylic groups in their structure (bifunctionalized) act as reticulating agents. The monomers giving rise to these units are preferably ethylene glycol dimethacrylate, 1,3-butanediol diacrylate, 1,4-butanediol diacrylate, 1,6-hexanediol diacrylate, ethylene glycol diacrylate, fluorescein O,O'-diacrylate, glycerol 1,3-diglycerolate diacrylate, pentaerythritol diacrylate monostearate, 1,6-hexanediol ethoxylate diacrylate, 3-hydroxy-2,2-dimethylpropyl 3-hydroxy-2,2-dimethylpropionate diacrylate, bisphenol A ethoxylate diacrylate, di(ethylene glycol) diacrylate, neopentyl glycol diacrylate, poly(ethylene glycol) diacrylate, poly(propylene glycol) diacrylate, propylene glycol glycerolate diacrylate, tetra(ethylene glycol) diacrylate, 1,3-butanediol dimethacrylate, 1,4-butanediol dimethacrylate, 1,6-hexanediol dimethacrylate, bisphenol A dimethacrylate, diurethane dimethacrylate, ethylene glycol dimethacrylate, fluorescein O,O'-dimethacrylate, glycerol dimethacrylate, bisphenol A ethoxylate dimethacrylate, bisphenol A glycerolate dimethacrylate, di(ethylene glycol) dimethacrylate, poly(ethylene glycol) dimethacrylate, poly(propylene glycol) dimethacrylate, tetraethylene glycol dimethacrylate, tri(ethylene glycol) dimethacrylate, triethylene glycol dimethacrylate, poly(lauryl methacrylate-co-ethylene glycol dimethacrylate), poly(methyl methacrylate-co-ethylene glycol dimethacrylate).

The proportion of acrylic or methacrylic units having a glycidyl group in their structure is preferably comprised

between 0.1 and 10% weight/weight of the total of the components of the base framework; the proportion of bifunctionalized acrylic or methacrylic units is preferably comprised between 0.1% and 10% weight/weight; and the proportion of acrylic or methacrylic units having an acrylic or methacrylic group in their structure is preferably comprised between 80% and 99.8% weight/weight.

In a preferred embodiment, the mixture of step a. is carried out in the absence of cyclodextrin, and more particularly of monomers comprising cyclodextrin or a portion derived from same.

In a preferred embodiment, the mixture of step a. or the monomers of the mixture of step a. only consist of acrylic or methacrylic monomers having/comprising a glycidyl group, monofunctionalized acrylic or methacrylic monomers, and bifunctionalized acrylic or methacrylic monomers.

Preferably, step a. is carried out in the presence of a polymerization initiator, for example azobisisobutyronitrile (AIBN). Polymerization can be initiated by heating the mixture of monomers or by exposing said mixture to ultraviolet-visible radiation.

The process of polymerization can be performed in molds having suitable dimensions for providing the hydrogels with the required shape. Therefore, in one embodiment the polymerization reaction is carried out in a mold providing the hydrogel with a contact lens shape. In a preferred embodiment, the mold is a mold of glass, polypropylene, polyethylene, or polytetrafluoroethylene.

In step b., the hydroxyl groups of the units of cyclodextrin react with the glycidyl groups present in the base framework of the hydrogel, giving rise to ether bonds.

The cyclodextrin of step b. is preferably a cyclodextrin solution, and even more preferably it is an alkaline cyclodextrin solution (basic pH). Alkaline solution is understood to mean that solution with a pH greater than 7, preferably a pH of 9 to 13.5.

In a preferred embodiment, the cyclodextrin solution has a concentration of cyclodextrin between 1% and 5% by weight of the solution.

In a preferred embodiment, the reaction of the base framework with cyclodextrin is by dipping the base framework in a cyclodextrin solution, preferably in an alkaline cyclodextrin solution.

In specific embodiments, the cyclodextrin used in the method of the invention is any of those described above in the embodiments corresponding to the hydrogel.

The ALA is incorporated in the three-dimensional acrylic hydrogel with pendant cyclodextrins by submerging it in a solution or in a suspension of the demulcent. The ALA forming inclusion complexes with the cyclodextrins can also be incorporated before carrying out the reaction of the base framework.

The concentration of ALA used will vary depending on the use to be given to the hydrogel or contact lens. In a preferred embodiment, the concentration of ALA used for preparing the hydrogel or lens is an ALA excess, that is: a higher molar amount of ALA with respect to the other components used for preparing the hydrogel, and more specifically with respect to the cyclodextrin; or the amount necessary to assure the maximum possible load of ALA in the hydrogel or contact lens; or the amount necessary to assure maximum possible occupation of ALA in the cyclodextrins in the hydrogel or contact lens.

In cases where an additional active agent or agents are included in the hydrogel, as described above, the active agent or agents are added during the method of preparation of the hydrogel as described above for ALA. The additional active agent or agents may be added to the ALA simultaneously or at different times.

In one embodiment, the hydrogels resulting from any of the preceding embodiments are washed and, optionally, dried.

The method is advantageous since it does not require previously obtaining a vinyl, acrylic, or methacrylic cyclodextrin monomer having polymerizable groups.

If the polymerization reaction has not been carried out in a mold providing the hydrogel with a contact lens shape, the contact lens can be prepared by means of lathe-cutting the hydrogel, or by means of molding the hydrogel, particularly by means of centrifugal molding or by means of cast molding, or by means of combinations of these techniques.

In another aspect, the present invention relates to hydrogels obtainable by means of a method according to any of the embodiments described above. A hydrogel in which the cyclodextrin has not interfered in the polymerization and crosslinking process is thereby obtained, and therefore base frameworks in which the cyclodextrin units are structural links of the chains forming the hydrogel are not obtained. The base frameworks are thereby prevented from having high rigidity as cyclodextrin acts like a reticulating agent.

Ocular use

In another aspect, the invention relates to the medical use of a hydrogel of the present invention, and more specifically of a contact lens of the present invention.

ALA is administered to the eye, and more specifically to the surface of the front part of the eye (or anterior segment of the eye), and even more particularly to the cornea, through the hydrogel or contact lens of the present

invention. Furthermore, the hydrogel or contact lens of the present invention advantageously allows said administration to be controlled/sustained and not immediate.

In one embodiment, the invention relates to a hydrogel of the present invention, and more specifically to a contact lens of the present invention, for use in medicine. More particularly, the use in medicine is a use in ophthalmology.

In the context of the present invention, the term "ophthalmology" refers to the branch of medicine relating to diseases of the eyes.

In a more specific embodiment, the invention relates to a hydrogel of the present invention, and more specifically to a contact lens of the present invention, for use in the treatment or prevention of dryness of the eye.

As they are used herein, the terms "treat," "treating," "treatment," and the like include the eradication, elimination, reversal, alleviation, modification, or control of the pathological condition.

As they are used herein, the terms "prevention," "preventing," "preventive," "prevent," prophylaxis, and the like refer to impeding, minimizing, or hindering the onset or the development of the pathological condition.

Dryness of the eye is a condition in which a person's eye is not exposed to tears in a sufficient amount or with a sufficient quality to lubricate and nourish the eye. Tears are needed to maintain the health of the front surface of the eye and to provide clear vision. With each blink, tears are spread over the front surface of the eye, known as the cornea. Tears provide lubrication, reduce the risk of eye infection, clean foreign matter out of the eye, and keep the surface of the eyes smooth and clear.

Dryness of the eyes may occur for a series of reasons.

Firstly, it may occur when the level of tear evaporation or draining in the eye exceeds the level of tear production, such that the eye is not exposed to a sufficient amount of tears. Likewise, it may occur when tear production itself is insufficient.

In other cases, it may occur when the tears are of a poor quality, i.e., their composition is changed and does not serve to perform their natural ocular functions. For example, tears comprise oil the function of which is to form a smooth layer of oil over the surface of the eye preventing the evaporation of water on the ocular surface. If the tear contains low levels of oil, or the components of the oil are not the correct components, the formation of the layer of oil will be affected and may give rise to excessive levels of water evaporation.

As mentioned above, ALA produces an increase in tear production. Therefore, the present invention particularly relates to the hydrogel of the present invention, and more specifically to a contact lens of the present invention, for use in the treatment or prevention of dryness of the eyes characterized by exposure of the eye, and more specifically of the surface of the front part of the eye (or anterior segment of the eye), and even more particularly of the cornea, to a low amount of tears. In more particular embodiments, the low amount of tears is due to insufficient tear production, or to excessive tear evaporation or draining. In the most preferred embodiment, dryness of the eyes is due to insufficient tear production. In a particular embodiment, dryness of the eyes is not due to a viral, bacterial, or fungal infection.

In one embodiment, the present invention particularly relates to the hydrogel of the present invention, and more specifically to a contact lens of the present invention, for use in the treatment or prevention of dry eyes through the increase in peroxidase activity in the eye.

In a preferred embodiment, the dryness of the eyes is dry eye syndrome, also known as keratoconjunctivitis sicca. In the context of the present invention, dry eye syndrome or keratoconjunctivitis sicca is understood to have the meaning given in the International Classification of Diseases-10 (ICD-10) of the World Health Organization, namely under code H19.3.

Another common cause of the onset of dry eye is the use of contact lenses, which disrupt the natural renewal of tear fluid. All this determines that many contact lens users, especially those around 40 years of age, feel a high degree of discomfort when they use contact lenses and opt to not use them. The incorporation of ALA into contact lenses allows preventing the onset of discomfort and improving user comfort by increasing tear production.

Therefore, in a preferred embodiment the dryness of the eyes is due to or is worsened by the use of contact lenses, more particularly the use of contact lenses which do not comprise demulcent, and even more particularly the use of contact lenses which do not comprise ALA. In particular, this use of contact lenses which causes or worsens the dryness of the eyes is for correcting vision.

Likewise, the invention therefore relates to the use of the hydrogel or the contact lens of the invention to treat or prevent the discomfort associated with the use of contact lenses, more particularly with the use of contact lenses which do not comprise demulcent, and even more particularly with the use of contact lenses which do not comprise ALA. In particular, this use of contact lenses which causes discomfort is for correcting vision.

In a particular embodiment, the contact lens of the present invention is used for the purposes described above and furthermore for correcting user's/patient's vision.

The incidence of dry eye syndrome increases with age as tear-generating cells become atrophied. In people over the age of 70, the incidence is close to 100%.

Therefore, in a preferred embodiment any of the uses described above are in patients 40 years of age or older, preferably 60 years of age or older, and even more preferably 70 years of age or older.

The invention likewise relates to the use of a hydrogel of the present invention, and more specifically a contact lens of the present invention, for the preparation of a medicinal product intended for any of the previously mentioned uses, more specifically for the treatment or prevention of dryness of the eye, or for the treatment or prevention of the discomfort associated with the use of contact lenses.

The invention likewise relates to a method for the prevention or treatment of dryness of the eye or of the discomfort associated with the use of contact lenses, which method comprises administering to a patient suffering from said dryness or discomfort a hydrogel of the present invention, and more specifically a contact lens of the present invention.

The concentration of ALA in the hydrogel or in the contact lens will vary depending on the nature and severity of dryness of the eye to be treated, or on the severity of the symptoms derived from dryness of the eyes such as ocular irritation or inflammation. In one embodiment, the concentration of ALA in the hydrogel is at least 25 mg of ALA for every 1 g of dry mass of hydrogel. In another embodiment, it is at least 35 mg of ALA for every 1 g of dry mass of hydrogel. In another embodiment, it is at least 45 mg of ALA for every 1 g of dry mass of hydrogel. In another embodiment, it is at least 55 mg of ALA for every 1 g of dry mass of hydrogel. In another embodiment, it is at least 65 mg of ALA for every 1 g of dry mass of hydrogel. In another embodiment, it is between 25 and 65 mg of ALA for every 1 g of dry mass of hydrogel. In another embodiment, it is between 35 and 65 mg of ALA for every 1 g of dry mass of hydrogel. In another embodiment, it is between 45 and 65 mg of ALA for every 1 g of dry mass of hydrogel. In another embodiment, it is between 55 and 65 mg of ALA for every 1 g of dry mass of hydrogel.

In one embodiment, the contact lens is used for 1 day and then disposed of.

The invention is described below by means of the following examples which must be considered as being merely illustrative and by no means limiting of the scope of the present invention.

## Examples

The following materials were used in the examples below: 2-hydroxyethyl methacrylate (HEMA), 3-methyl benzoic acid (3-MBA), and dimethylformamide (DMF) by Merck (Hohenbrunn, Germany); 2,2'-azobis(2-methylpropionitrile) (AIBN) and α-lipoic acid (ALA, 206.33 g/mol) by Acros Organics (New Jersey, USA); 98% ethylene glycol dimethacrylate (EGDMA) and phosphate-buffered saline (PBS) by Sigma-Aldrich (Steinheim, Germany); glycidyl methacrylate (GMA) by Polysciences Inc. (Warrington, UK); α-cyclodextrin (α-CD, 973 Da, Cavamax W6) by Wacker Química Ibérica S.A. (Barcelona, Spain); β-cyclodextrin (β-CD, 1135 Da, Kleptose®) by Roquette Frères (Lestrem, France); NaOH by VWR Chemicals (Leuven, Belgium); penicillin/streptomycin (10,000 U/ml) by Thermo Fisher Scientific (Waltham, USA), and water purified by reverse osmosis (resistivity > 18MΩ·cm, MilliQ, Millipore® Spain). Simulated tear fluid (STF) prepared with the following composition: 6.78 g/L NaCl by VWR Chemicals (Leuven, Belgium); 2.18 g/L $NaHCO_3$ and 1.38 g/L KCl by Panreac (Barcelona, Spain); and 0.084 g/L $CaCl \cdot 2H_2O$ by Merck (Darmstadt, Germany), with pH 7.8.

The hydrogels of the present invention can be prepared as described in document EP 2 314 640 A1.

## Synthesis of acrylic hydrogels

A mixture of ethylene glycol dimethacrylate (EGDMA; 0.0714 mL, 8 mM) and AIBN (0.0741 g, 10 mM) was prepared in 45 mL of hydroxyethyl methacrylate (HEMA). The mixture was divided into two aliquots of 15 and 30 mL, to which 0 and 1.594 mL of glycidyl methacrylate (GMA) were added, respectively, to have a GMA content of 0 (control, without CD) and 400 mM. Next, with the aid of a syringe, 5 mL of these solutions were introduced in different glass molds. These molds were taken to an air flow oven at 50°C, where they were kept for 12 h. Then, the temperature was increased to 70°C and left for another 24 h. After that time lapsed, the hydrogel sheets were removed from the molds, submersed for 15 min in boiling water, and cut into a disc shape using a punch 10 mm in diameter. The obtained discs were submersed in water for 24 h. Next, those discs were transferred to a 0.9% NaCl solution and were maintained for another 24 h. Finally, they were kept in water for 72 h until use.

## Cyclodextrin anchoring

100 mL of a 0.5 M NaCl solution were prepared and 100 mL of DMF were added to it. This 0.5 M NaCl:DMF (50:50 v/v) solution was divided into two aliquots of 100 mL in Pyrex flasks, NaOH (3%) was added to them and they were heated at 80°C. α-CD was dissolved in one of the aliquots and β-CD was dissolved in the other aliquot. Moist hydrogel discs with 400 mM GMA were incorporated into the solutions and they were kept under magnetic stirring for 24 h in a temperature-controlled bath at 80°C.

In order to determine the amount of CD loaded in the discs, three dry discs of each type of hydrogel (with α-CD, and with β-CD) were taken, weighed, and submersed, separately, in 10 mL of 3-MBA 0.5 mg/mL solution. They were kept for 48 h protected from light. Next, the concentration of 3-MBA remaining in the solution was determined by measuring absorbance at 281 nm (Agilent 8453 UV/VIS spectrophotometer, Germany). The amount of 3-MBA taken up

by the hydrogel discs was calculated by the difference between the initial amount and the final amounts in the solution (determined from the calibration line of 3-MBA in water), and referred to the unit of mass of dry hydrogel, using the equation:

$$3 - MBA\ incorporated\ \left(\frac{mg}{g}\right) = \frac{\left(C_{initial} - C_{final}\right) * Volume}{weight\ of\ dry\ hydrogel\ (g)}$$

In this equation, $C_{initial}$ and $C_{final}$ represent the initial and final concentrations of 3-MBA in the aqueous solution. The amount of 3-MBA incorporated by the effect of CDs was estimated by the difference between the amount loaded in the hydrogel discs with CD and the amount taken up by the discs without CD.

It was established that the load of CD in the discs was 31±1 mg/g.

Demulcent loading

Next, a different demulcent was loaded in each disc functionalized with CD.

*Polysorbate 80 (Sigma Aldrich)*

The weighed hydrogel discs were placed in 5 or 10 mL of aqueous polysorbate 80 solutions (0.2; 0.5 or 1 mg/mL) and kept at 20°C under magnetic stirring (80 rpm) for 24 hours. Absorbance of the medium was monitored at 234 nm (Agilent mL8543 UV/Vis spectrophotometer, Germany). The amount of loaded polysorbate 80 was estimated from the difference between the amounts in the loading medium before and after the loading tests.

*Polyethylene glycol 4000 (Sigma Aldrich)*

The weighed hydrogel discs were placed in 5 or 10 mL of aqueous PEG4000 solutions (0.1 mg/mL), kept at 20°C under magnetic stirring (80 rpm) for 48 hours. Aliquots of loading medium were analyzed by means of HPLC. The loaded amount of PEG4000 was estimated from the difference between the amounts in the loading medium before and after the loading tests.

*α-lipoic acid*

The weighed hydrogel discs were introduced in vials containing 10 mL of aqueous ALA solution (saturated, ~0.8 mg/mL). The vials remained under oscillating agitation (150 osc/min) at 20°C. At times 24 and 48 h, a sample of 3 mL of the loading solution was taken and absorbance was measured at 334 nm (Agilent 8453 UV/VIS, Germany). The amount of ALA loaded for every hydrogel was calculated by the difference between the initial content in the loading solution and the remaining content at the end of the test.

The loading results, measured in amount of demulcent for every gram of dry mass of hydrogel, are shown below:

| Demulcent | 24 h | 48 h |
|---|---|---|
| Polysorbate 80 | 1 mg/g | |
| Polyethylene | | 0.5 mg/g |
| glycol 4000 | | |
| α-lipoic acid | 57 mg/g | 58 mg/g |

As can be observed, the ALA load was of a magnitude that was surprisingly much higher than that of other demulcents.

In light of these results, additional tests were carried out with the hydrogels loaded with ALA.

Transfer of α-lipoic acid

The discs coming from the loading test were rinsed with milliQ® water, the excess water was removed from the surface with filter paper, and each disc was arranged in a vial with 3 mL of simulated tear fluid (STF) and the vials were kept under oscillating agitation (150 rpm) at 35°C. At pre-established times (0.5; 1; 1.5; 2; 2.5; 3; 3.5; 4; 6; 8; 24;

and 48 h), 2.5 mL of the transfer medium were taken, absorbance was measured at 334 nm (Agilent 8453 UV/VIS, Germany) and the sample was integrated into the vial of origin. To calculate the amount of ALA transferred for each hydrogel (with α-CD, with β-CD, and without CD), the concentration in the transfer medium was calculated from the absorbances using the corresponding calibration line of ALA in STF, and next the concentrations were transformed into amounts multiplying by the volume of the transfer medium.

The percentage of transfer was similar for the three types of hydrogel (see Figure 1). After an initial burst around 30%, the transfer was prolonged for about 6 hours. These results under sink conditions suggest that under dynamic conditions of the eye, the transfer would be prolonged for even more time.

Preparation of contact lenses loaded with α-lipoic acid

Dried contact lenses were prepared from a hydrogel according to the invention. The preparation was by means of lathe-cutting or by means of molding the hydrogel. The hydrogel not functionalized with CD contained 400 mM GMA and was functionalized with β-CD (NaCl/NaOH/DMF medium at 60°C for 2 hours).

The weighed contact lenses were introduced in 4 mL of aqueous ALA solution (saturated, ~0.8 mg/mL). The loaded amount of ALA was estimated from the difference between the initial amount of the demulcent in the solution and the amount remaining after loading, determined spectrophotometrically.

The ALA load, measured as the amount of demulcent for every gram of dry mass of lens, reached an average of about 50 mg/g (and in no case was it less than 47 mg/g), taking into account both the lenses prepared by means of lathe-cutting and those prepared by means of molding. It is thereby demonstrated that the preparation of contact lenses with the surprising high amounts of ALA observed with the hydrogels is likewise possible.

Corneal permeability study

*a. hydrogels*

Fresh cow eyes were collected at the slaughterhouse and transported submerged in PBS (phosphate-buffered saline) with added antibiotics (penicillin at 100 IU/mL and streptomycin at 100 μg/ml) in an ice bath. Nine corneas were isolated with 2-3 mm of surrounding sclera and cleaned with 0.9% NaCl. Each cornea was mounted in a Franz-Chien type vertical diffusion cell, separating the donor from the receiver chamber. The chambers were filled with bicarbonate buffer pH 7.8. The diffusion cells were arranged in a temperature-controlled bath at 37°C and a magnet was placed in the receiver chamber to keep it under moderate stirring. After 1 h has lapsed, the buffer was removed from the donor chamber.

Hydrogel discs with β-CD loaded with ALA were placed on three corneas, and discs without CD loaded with ALA were placed on another three corneas. Next, 0.5 mL of bicarbonate buffer were added to the 6 donor chambers which contained discs. 2 mL of ALA solution (0.81 mg/mL) were added on the three remaining corneas as a control. The donor chambers were covered with parafilm. 1 mL samples were taken from the receiver chambers at 1 h intervals, making use of a syringe with needle, and the same volume of bicarbonate buffer was replaced each time, being careful to eliminate air bubbles.

The samples taken from the receiver chambers were filtered through nylon membranes (0.45 μm) and measured with HPLC equipment (Waters 717 Autosampler, Waters 600 Controller, 996 Photodiode Array Detector) conditioned with a C18 column (Waters Symmetry C18 5 μm; 3.9 x 150 mm) and Empower 2 as software. The mobile phase consisted of methanol:buffer solution:acetonitrile (51:41:8; $KH_2PO_4$ buffer adjusted to pH 3-3.1 with o-phosphoric acid) at 0.8 mL/min and 35°C. The injected volume was 50 μL, and the ALA was quantified at 198 nm (the wavelength showing the highest intensity). The calibration line of ALA in aqueous phase was prepared in the range of concentrations comprised between 0.005 and 0.50 mg/mL. The ALA retention time was 5.1 min. The ALA content of the samples was calculated from the calibration curve.

After 6 h of exposure, the concentration of ALA in the 0.5 mL of medium in which the discs were submerged was determined. The discs and the ALA solution were removed from the donor chamber and the corneas were washed 3 times with 0.9% NaCl. The surface of the cornea exposed to the antioxidant agent was cut with a punch 11 mm in diameter and introduced in a 15 mL Falcon® tube which contained 3 mL of ethanol:water extraction medium (50:50 v/v). The samples were closed with a screw-on cap and sonicated in an ultrasound bath (Branson 3510, Branson Ultrasonics, Danbury, CT, USA) for 99 min at 37°C. At the end of the sonication cycle, the samples were centrifuged a 1000 rpm for 5 min at 25°C to separate residues from the tissue. The supernatant liquid was filtered (nylon membrane 0.45 μm and 13 mm in diameter), and the filtrate was decanted to 2 mL Eppendorf® tubes and centrifuged at 14,000 rcf for 10 min to remove insoluble proteins. The amount of ALA extracted from the corneas was quantified by HPLC. To quantify the amount of ALA remaining in the discs, they were submersed in 2 mL of ethanol:water (50:50 v/v) and kept in an oscillating agitator at 200 rpm for 24 h at 20°C. The samples were filtered and analyzed by HPLC. The ALA remaining in the solution

placed in the donor chamber was quantified from the absorbance values measured at 334 nm (Agilent 8453 UV/VIS spectrophotometer, Germany).

The amount of ALA that passed through the cornea to the receiving medium is shown in Figure 2.

The amounts of ALA initially applied in the cornea, the amounts which have permeated the cornea, the amounts remaining in the donor chamber, the amounts accumulated in the cornea, and the amounts remaining in the hydrogel discs, are shown in Figure 3.

The results clearly show the capacity of the ALA loaded in hydrogels according to the present invention to act on and even permeate the cornea. Furthermore, a considerable improvement is observed with respect to hydrogels not functionalized with CD, giving rise to higher amounts of corneal and transcorneal demulcent.

*b. contact lenses*

Fresh cow eyes were collected at the slaughterhouse and transported submerged in phosphate-buffered saline (PBS) in an ice bath. Six corneas were isolated with 2-3 mm of surrounding sclera and cleaned with 0.9% NaCl. Each cornea was mounted in a Franz-Chien type vertical diffusion cell, separating the donor from the receiver chamber. The chambers were filled with bicarbonate buffer, pH=7.8, placed under temperature-controlled conditions in a bath at 35°C, and a magnet was placed in the receiver chamber to keep it under moderate stirring. After one hour has lapsed, the buffer was removed from the donor chamber.

Lenses functionalized with β-CD, and loaded with ALA, were placed on 3 corneas, and 0.5 ml of bicarbonate buffer were added. 0.5 ml of a solution of ALA (2.2 mg/ml) in bicarbonate buffer were added to the 3 other corneas.

1 ml samples were taken from the receiver chambers at determined times, replacing them with the same volume of the bicarbonate buffer solution.

The samples taken from the receiver chamber were filtered through 0.45 μm nylon membranes and measured in HPLC equipment (Waters 717 Autosampler, Waters 600 Controller, 996 Photodiode Array Detector) conditioned with a C18 column (Waters Symmetry C18 5 μm, 4.6 x 250 mm, batch 029936202) under temperature-controlled conditions at 35°C and Empower 2 as a data processing program.

The mobile phase was a solution of methanol:phosphate buffer solution, pH=3.05:acetonitrile in a proportion of 51:41:8 at a flow of 1.2 ml/min. 50 μL of each sample and of the solutions of the calibration lines, quantified at 215 nm were injected.

After 6 hours of exposure, the solution was removed from the donor chamber and the corneas were washed with 0.9% NaCl. The exposed surface of the cornea was cut with a punch 12 mm in diameter and weighed. It was introduced in a 15 ml tube with 3 ml of ethanol:water (50:50 v/v) extraction medium. They were closed with a screw-on cap and were taken to an ultrasound bath for 99 minutes at 37°C. After this process, they were centrifuged at 1000 rpm for 5 minutes at 25°C. The supernatant liquid was filtered, decanted into 2 ml Eppendorf tubes, and centrifuged at 14000 rpm for 20 minutes to remove insoluble proteins. The amount of ALA extracted from the corneas was quantified by HPLC.

To assess the amount of ALA remaining in the contact lenses, they were submersed in 5 ml Eppendorf tubes, 2 ml of ethanol:water (50:50 v/v) were added, and they were kept under oscillating agitation for 24 hours at 200 rpm, after which time the samples were filtered and analyzed by HPLC.

The ALA remaining in the solution placed in the donor chamber was also quantified by means of the HPLC technique.

The following results were observed:

| Permeate (μg/cm$^2$) Lenses with CD | | | | | |
|---|---|---|---|---|---|
| Time (min) | Cell 1 | Cell 2 | Cell 3 | Mean | STD |
| 0 | 0 | 0 | 0 | 0 | 0 |
| 10 | 0 | 0 | 0 | 0 | 0 |
| 20 | 0 | 0 | 0 | 0 | 0 |
| 30 | 0 | 0 | 0 | 0 | 0 |
| 45 | 0 | 0 | 0 | 0 | 0 |
| 60 | 0 | 0 | 0 | 0 | 0 |
| 120 | 7.37 | 13.11 | 8.11 | 9.53 | 3.12 |
| 180 | 21.53 | 26.06 | 17.64 | 21.75 | 4.21 |
| 240 | 35.38 | 37.08 | 30.40 | 34.28 | 3.47 |

(continued)

| Permeate (μg/cm²) Lenses with CD | | | | | |
|---|---|---|---|---|---|
| Time (min) | Cell 1 | Cell 2 | Cell 3 | Mean | STD |
| 300 | 47.68 | 43.37 | 34.02 | 41.69 | 6. 98 |
| 360 | 60.65 | 52.19 | 40.40 | 51.08 | 10.17 |

| Permeate (μg/cm²) Solution of ALA in Buffer | | | | | |
|---|---|---|---|---|---|
| Time (min) | Cell 4 | Cell 5 | Cell 6 | Mean | STD |
| 0 | 0 | 0 | 0 | 0 | 0 |
| 10 | 0 | 0 | 0 | 0 | 0 |
| 20 | 0 | 0 | 0 | 0 | 0 |
| 30 | 0 | 0 | 0 | 0 | 0 |
| 45 | 0.00 | 5.66 | 5.14 | 3.60 | 3.13 |
| 60 | 0.00 | 9.35 | 7.56 | 5. 64 | 4.96 |
| 120 | 14.63 | 28.54 | 28.92 | 24.03 | 8.15 |
| 180 | 23.04 | 46.54 | 47.54 | 39.04 | 13.86 |
| 240 | 39.55 | 77.56 | 67.24 | 61.45 | 19.65 |
| 300 | 50.75 | 94.70 | 86.77 | 77.41 | 23.42 |
| 360 | 75.42 | 106.10 | 115.19 | 98.90 | 20.84 |

The results are shown graphically in Figure 4.

As can be observed, the results clearly show the capacity of the ALA loaded in the contact lenses according to the present invention to act on and even permeate the cornea.

It was additionally confirmed that the correlation between the amount of permeated ALA and the lapsing of time is linear, and the ALA flow (in $\mu g/cm^2$ x s) was determined from said linear correlation (as shown in Figures 5 and 6). The following flows were observed:

| | Flow ($\mu g/cm^2$ x min) | Flow ($\mu g/cm^2$ x s) |
|---|---|---|
| ALA β-CD lenses | 0.1717 | 0.00286 |
| ALA solution | 0.3135 | 0.00523 |

Visible light transmission

The transmittance percentage of the discs of each type of hydrogel (with α-CD and with β-CD) was measured before and after loading ALA and then hydrated in water or in phosphate buffer, pH 7.4, using a spectrophotometer (Agilent 8453 UV/VIS, Germany). Each disc was introduced in the quartz cuvette (1 cm light path) and contacted with the inner face perpendicular to the incident light beam. The cuvette was filled with water or buffer. Transmittance was recorded between 190 and 800 nm. The tests were carried out in triplicate. All the discs presented transmittance values higher than 90% between 400 and 600 nm. Furthermore, the transmittance of the discs was not affected after being loaded with ALA.

## Claims

1. Hydrogel in the form of a three-dimensional network, **characterized in that** it comprises crosslinked acrylic or methacrylic chains, wherein the chains comprise alkyl groups to which cyclodextrins are attached by means of an

ether bond; and wherein the hydrogel further comprises α-lipoic acid, or a pharmaceutically acceptable salt thereof.

2. Hydrogel according to claim 1, **characterized in that** the acrylic or methacrylic chains comprise: acrylic or methacrylic units comprising an alkyl ether group, bifunctionalized acrylic or methacrylic units, and monofunctionalized acrylic or methacrylic units.

3. Hydrogel according to claim 2, **characterized in that** the proportion by weight of the acrylic or methacrylic units comprising an alkyl ether group is between 0.1% and 10% with respect to the weight of the hydrogel.

4. Hydrogel according to claims 2 and 3, **characterized in that** the proportion by weight of the bifunctionalized acrylic or methacrylic units is between 0.1% and 10% with respect to the weight of the hydrogel.

5. Hydrogel according to any of the preceding claims, wherein the cyclodextrin is α- or β-cyclodextrin.

6. Hydrogel according to any of the preceding claims, comprising at least one active agent in addition to α-lipoic acid.

7. Hydrogel according to claim 6, wherein the active agent is a demulcent.

8. Hydrogel according to claim 7, wherein the demulcent is selected from the group consisting of cellulose derivatives, dextran, gelatin, and polyols.

9. Hydrogel according to claim 6, wherein the active agent is an anti-inflammatory agent.

10. Hydrogel according to claim 9, wherein the anti-inflammatory agent is selected from the group consisting of ibuprofen, ketoprofen, flurbiprofen, fenoprofen, naproxen, piroxicam, tenoxicam, isoxicam, meloxicam, indomethacin, aceclofenac, and diclofenac.

11. Method for obtaining a hydrogel as defined in any of claims 1 to 10, comprising the steps of:

a. polymerizing and crosslinking a mixture comprising i) monofunctionalized acrylic or methacrylic monomers, and ii) acrylic or methacrylic monomers comprising a glycidyl group, to form a base framework of a three-dimensional network; and
b. reacting cyclodextrin with the base framework;
wherein α-lipoic acid is added either to the hydrogel after step b., or to the cyclodextrin before carrying out step b.

12. Method according to claim 11, wherein the mixture of step a. further comprises monomers having two or more acrylic or methacrylic groups in their structure.

13. Method according to claim 11 or 12, wherein the monofunctionalized acrylic or methacrylic monomers are selected from the group consisting of hydroxyethyl methacrylate, 1-(tristrimethylsiloxysilylpropyl)-methacrylate, methylmethacrylate, N,N-dimethylacrylamide, N,N-diethylacrylamide, methacrylic acid, acrylic acid, aminopropyl methacrylate, cyclohexyl methacrylate, and fluoro-siloxane acrylate.

14. Method according to any of claims 11 to 13, wherein the acrylic or methacrylic monomers comprising a glycidyl group are glycidyl acrylate or glycidyl methacrylate.

15. Method according to any of claims 12 to 14, wherein the monomers having two or more acrylic or methacrylic groups in their structure are selected from the group consisting of ethylene glycol dimethacrylate, 1,3-butanediol diacrylate, 1,4-butanediol diacrylate, 1,6-hexanediol diacrylate, ethylene glycol diacrylate, fluorescein O,O'-diacrylate, glycerol 1,3-diglycerolate diacrylate, pentaerythritol diacrylate monostearate, 1,6-hexanediol ethoxylate diacrylate, 3-hydroxy-2,2-dimethylpropyl 3-hydroxy-2,2-dimethylpropionate diacrylate, bisphenol A ethoxylate diacrylate, di(ethylene glycol) diacrylate, neopentyl glycol diacrylate, poly (ethylene glycol) diacrylate, poly(propylene glycol) diacrylate, propylene glycol glycerolate diacrylate, tetra(ethylene glycol) diacrylate, 1,3-butanediol dimethacrylate, 1,4-butanediol dimethacrylate, 1,6-hexanediol dimethacrylate, bisphenol A dimethacrylate, diurethane dimethacrylate, ethylene glycol dimethacrylate, fluorescein O,O'-dimethacrylate, glycerol dimethacrylate, bisphenol A ethoxylate dimethacrylate, bisphenol A glycerolate dimethacrylate, di(ethylene glycol) dimethacrylate, poly(ethylene glycol) dimethacrylate, poly(propylene glycol) dimethacrylate, tetraethylene glycol dimethacrylate, tri(ethylene glycol) dimethacrylate, triethylene glycol dimethacrylate, poly(lauryl methacrylate-co-ethylene glycol dimethacrylate), and poly(methyl methacrylate-co-ethylene glycol dimethacrylate).

**16.** Method according to any of claims 11 to 15, wherein the cyclodextrin is α- or β-cyclodextrin, or combinations thereof.

**17.** Method according to any of claims 11 to 16, wherein the mixture of step a. further comprises a polymerization initiator.

**18.** Method according to any of claims 11 to 17, wherein the reaction of the base framework of a three-dimensional network with cyclodextrin is by dipping the base framework in an alkaline cyclodextrin solution.

**19.** Hydrogel obtainable by means of a method as defined in any of claims 11 to 18.

**20.** Contact lens comprising a hydrogel as defined in any of claims 1 to 10 or 19.

**21.** Method for preparing a contact lens as defined in claim 20, which comprises forming the contact lens from the hydrogel as defined in any of claims 1 to 10 or 19 by means of lathe-cutting the hydrogel or by means of molding the hydrogel, or by means of a combination of these techniques.

**22.** Hydrogel as defined in any of claims 1 to 10 or 19, or contact lens as defined in claim 20, for use in medicine, preferably in ophthalmology.

**23.** Hydrogel or contact lens for use according to claim 22, for use in the treatment or prevention of dryness of the eye.

**24.** Hydrogel or contact lens for use according to claim 22, for use in the treatment or prevention of dry eye syndrome.

**25.** Hydrogel or contact lens for use according to any of claims 22 to 24, for use in the prevention or treatment of the discomfort associated with the use of contact lenses.

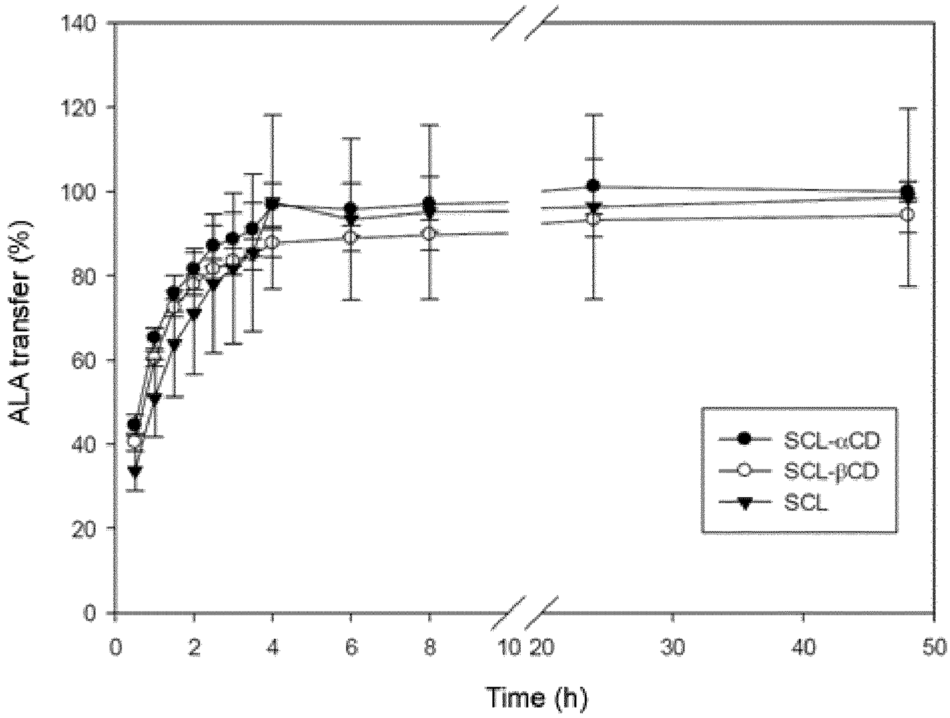
ALA transfer (%)
140
120
100
80
60
40
20
0
0
2
4
6
8
10
20
30
40
50
Time (h)
SCL-αCD
SCL-βCD
SCL

Fig. 1

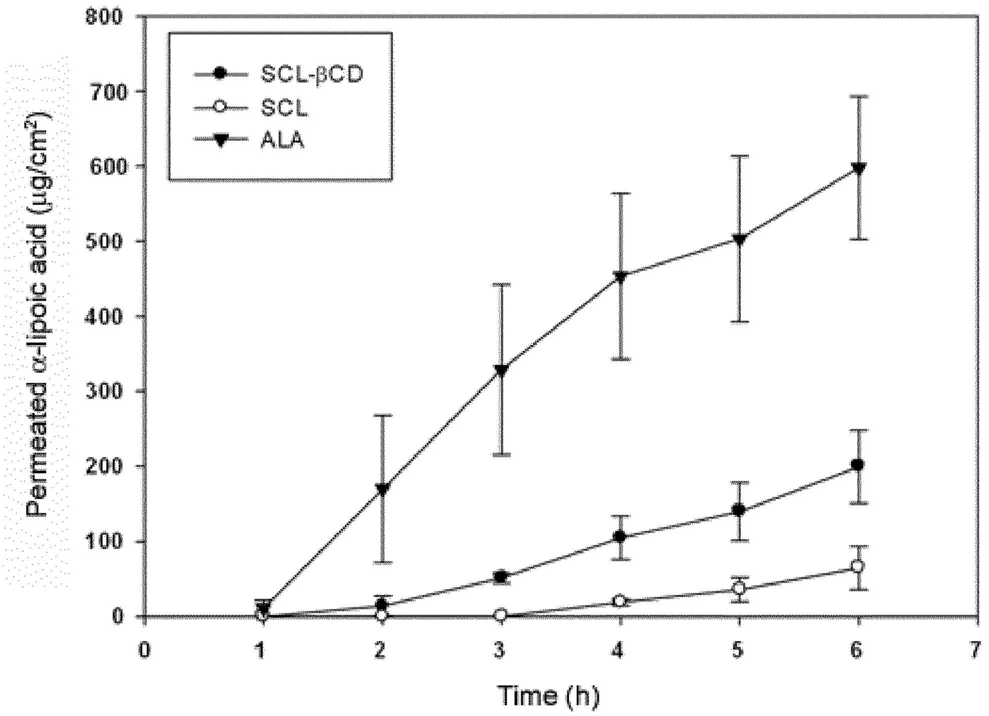
SCL-βCD
SCL
ALA
Permeated α-lipoic acid (μg/cm2)
Time (h)
800
700
600
500
400
300
200
100
0
0
1
2
3
4
5
6
7

Fig. 2

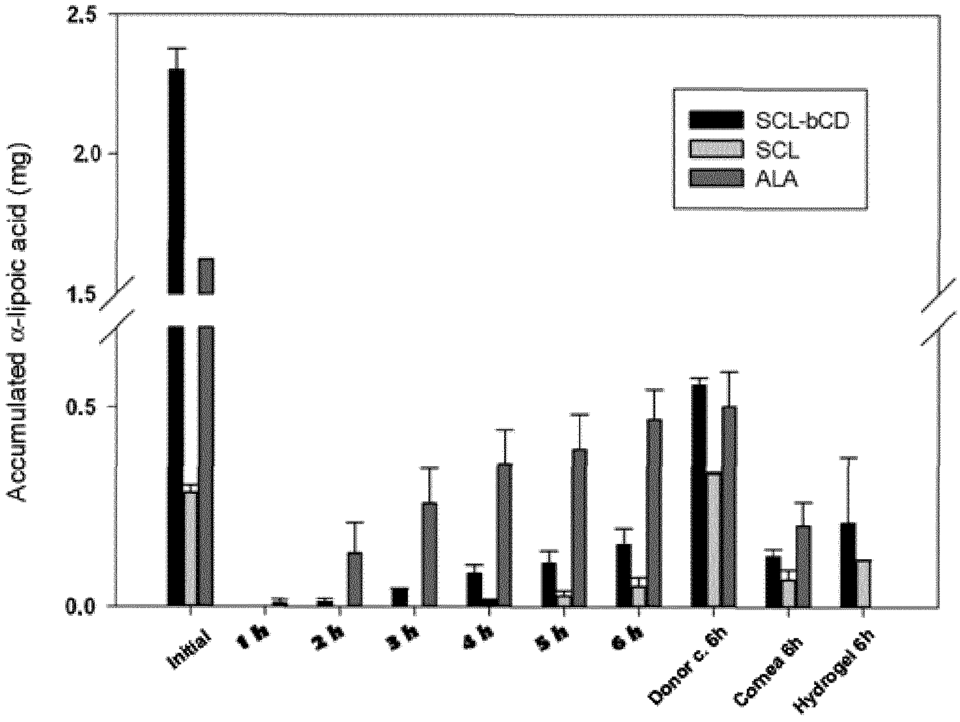
Accumulated α-lipoic acid (mg)
2.5
2.0
1.5
0.5
0.0
SCL-bCD
SCL
ALA
Initial
1 h
2 h
3 h
4 h
5 h
6 h
Donor c. 6h
Cornea 6h
Hydrogel 6h

Fig. 3

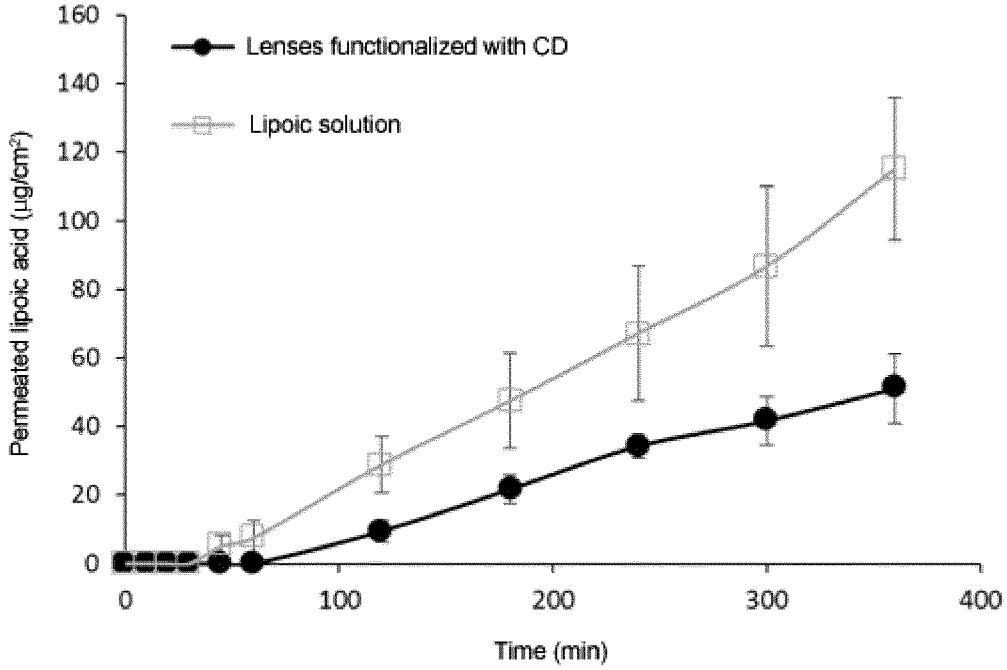
160
140
120
100
80
60
40
20
0
Lenses functionalized with CD
Lipoic solution
Permeated lipoic acid (µg/cm²)
0
100
200
300
400
Time (min)

Fig. 4

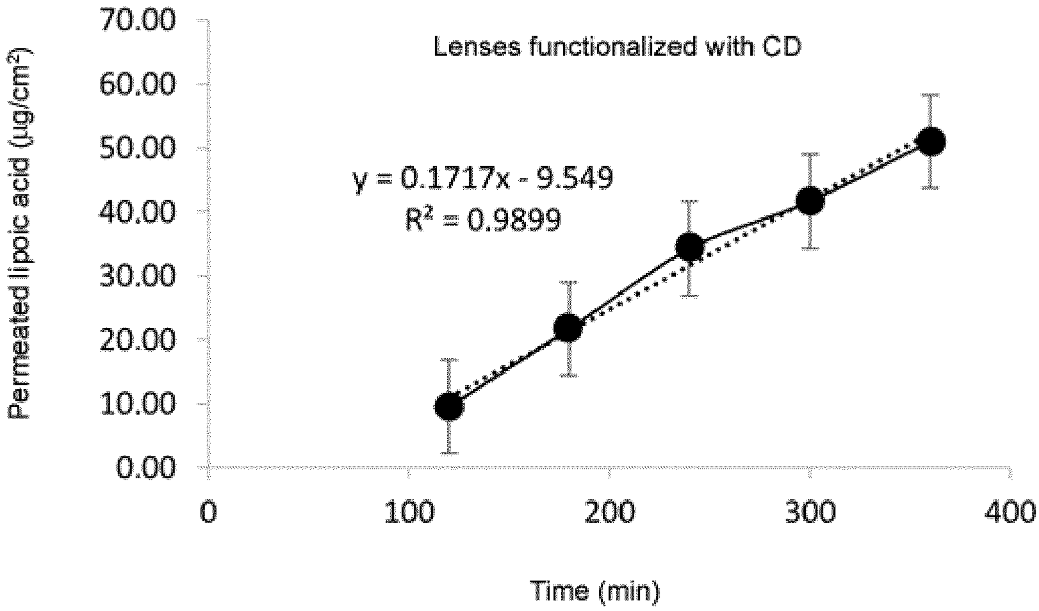
70.00
60.00
50.00
40.00
30.00
20.00
10.00
0.00
Lenses functionalized with CD
y = 0.1717x - 9.549
R² = 0.9899
Permeated lipoic acid (µg/cm²)
0
100
200
300
400
Time (min)

Fig. 5

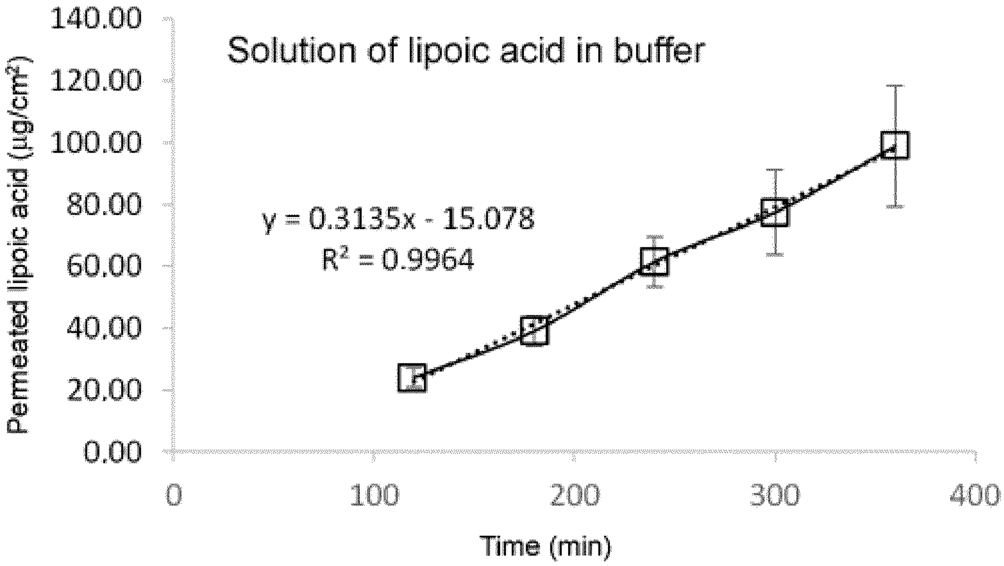
Solution of lipoic acid in buffer
y = 0.3135x - 15.078
R² = 0.9964
Permeated lipoic acid (µg/cm²)
140.00
120.00
100.00
80.00
60.00
40.00
20.00
0.00
0
100
200
300
400
Time (min)

Fig. 6

## INTERNATIONAL SEARCH REPORT

International application No.
PCT/ES2017/070282

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
C08F, C08B, C08J, G02C, G02B, A61K

Documentation searched other than minimum documentation to the extent that such documents are included in the fields searched

Electronic data base consulted during the international search (name of data base and, where practicable, search terms used)

EPODOC, INVENES, WPI, CAS, EMBASE, MEDLINE, BIOSIS, NPL, XPESP, XPESP2

C. DOCUMENTS CONSIDERED TO BE RELEVANT

| Category* | Citation of document, with indication, where appropriate, of the relevant passages | Relevant to claim No. |
|---|---|---|
| X | ES 2335958 A1 (UNIV SANTIAGO COMPOSTELA) 06/04/2010, page 3, lines 25-66; page 5, line 65-page 6, line 11; example 4. | 1-25 |
| A | ANDRADE ALEXEY, S. et al.; Alpha-lipoic acid restores tear production in an animal model of dry eye. EXPERIMENTAL EYE RESEARCH, 20140104 ACADEMIC PRESS LTD, LONDON. Tamm Ernst R; Dowling John E, 04/01/2014, Vol. 120, Pages 1 - 9, ISSN 0014-4835, <DOI: doi:10.1016/j.exer.2013.12.014> | 1-25 |

☒ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 10/11/2017 | (14/11/2017) |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>N. Vera Gutierrez<br><br>Telephone No. 91 3495544 |

Form PCT/ISA/210 (second sheet) (January 2015)

## INTERNATIONAL SEARCH REPORT

International application No.
PCT/ES2017/070282

| C (continuation). | DOCUMENTS CONSIDERED TO BE RELEVANT | |
|---|---|---|
| Category * | Citation of documents, with indication, where appropriate, of the relevant passages | Relevant to claim No. |
| A | WINTERTON LYNN, C. et al.; The elution of poly (vinyl alcohol) from a contact lens: the realization of a time release moisturizing agent/artificial tear.. Journal of biomedical materials research. Part B, Applied biomaterials United States Feb 2007. 31/01/2007, Vol. 80, Nº 2, Pages 424 - 432, ISSN 1552-4973 (Print), <DOI: pubmed:16838350> | 1-25 |
| A | US 2006100408 A1 (POWELL, P. M. et al.) 11/05/2006, paragraph [0002], claim 29. | 1-25 |

Form PCT/ISA/210 (continuation of second sheet) (January 2015)

**INTERNATIONAL SEARCH REPORT**

International application No.
PCT/ES2017/070282

**Box No. II Observations where certain claims were found unsearchable (Continuation of item 2 of first sheet)**

This international search report has not been established in respect of certain claims under Article 17(2)(a) for the following reasons:

1. [X] Claims Nos.: **22-25**
because they relate to subject matter not required to be searched by this Authority, namely:

Claims 22-25 concern a method for treatment of the human or animal body by therapy.
The search has been carried out on the basis of the possible effects of the composition.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest and, where applicable, the payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (January 2015)

## INTERNATIONAL SEARCH REPORT

International application No.

PCT/ES2017/070282

### CLASSIFICATION OF SUBJECT MATTER

***C08F220/06*** (2006.01)
***C08F220/10*** (2006.01)
***C08B37/16*** (2006.01)
***C08J3/075*** (2006.01)
***G02C7/04*** (2006.01)
***G02B1/04*** (2006.01)
***A61K31/385*** (2006.01)

Form PCT/ISA/210 (extra sheet) (January 2015)

## INTERNATIONAL SEARCH REPORT

Information on patent family members

International application No.
PCT/ES2017/070282

| Patent document cited in the search report | Publication date | Patent family member(s) | Publication date |
|---|---|---|---|
| ES2335958 A1 | 06.04.2010 | DK2314640T T3 | 12.10.2015 |
| | | JP2011529998 A | 15.12.2011 |
| | | JP5602734B B2 | 08.10.2014 |
| | | US2011200661 A1 | 18.08.2011 |
| | | US8951548 B2 | 10.02.2015 |
| | | WO2010018293 A1 | 18.02.2010 |
| | | EP2314640 A1 | 27.04.2011 |
| | | EP2314640 A4 | 14.12.2011 |
| US2006100408 A1 | 11.05.2006 | TW200728800 A | 01.08.2007 |
| | | KR20040094787 A | 10.11.2004 |
| | | KR100899364B B1 | 26.05.2009 |
| | | TWI325868B B | 01.08.2004 |
| | | TW200413419 A | 01.08.2004 |
| | | HK1079225 A1 | 25.03.2011 |
| | | AR038934 A1 | 02.02.2005 |
| | | AR070938 A2 | 12.05.2010 |
| | | JP2009221486 A | 01.10.2009 |
| | | JP5697317B B2 | 08.04.2015 |
| | | MXPA06011689 A | 07.03.2008 |
| | | US2006036052 A1 | 16.02.2006 |
| | | US7256246 B2 | 14.08.2007 |
| | | US2008015322 A1 | 17.01.2008 |
| | | US7816460 B2 | 19.10.2010 |
| | | US2003236376 A1 | 25.12.2003 |
| | | US6846892 B2 | 25.01.2005 |
| | | WO03077792 A2 | 25.09.2003 |
| | | WO03077792 A3 | 30.06.2005 |
| | | SG131862 A1 | 28.05.2007 |
| | | KR20070038923 A | 11.04.2007 |
| | | JP2007289641 A | 08.11.2007 |
| | | JP2005539098 A | 22.12.2005 |
| | | JP5220260B B2 | 26.06.2013 |
| | | EP1772767 A2 | 11.04.2007 |
| | | EP1772767 A3 | 08.07.2009 |
| | | EP1567567 A2 | 31.08.2005 |
| | | EP1567567 A4 | 28.02.2007 |
| | | CN1959469 A | 09.05.2007 |
| | | CN1697844 A | 16.11.2005 |
| | | CN1320004C C | 06.06.2007 |
| | | CA2562980 A1 | 07.04.2007 |
| | | CA2478307 A1 | 25.09.2003 |
| | | CA2478307 C | 29.05.2012 |
| | | BRPI0604242 A | 21.08.2007 |
| | | AU2006225292 A1 | 26.04.2007 |
| | | AU2003224658 A1 | 29.09.2003 |
| | | AU2003224658B B2 | 04.09.2008 |
| | | AR057150 A1 | 21.11.2007 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2314640 A1


### Non-patent literature cited in the description


- **ANDRADE et al.** *Exp Eye Res.,* 2014, vol. 120, 1-9